# EUROPEAN PATENT APPLICATION

(11) **EP 1 176 189 A1**
(43) Date of publication of application: **30.01.2002**
(21) Application number: 00202634.2
(22) Date of filing: 21.07.2000
(51) Int. Cl.: C12N 5/06, C12Q 1/68, A01K 67/027, A61K 35/12, A61F 2/00

(54) **Stem cell-like cells**

(71) Applicant: Fornix Biosciences N.V., 8243 PM Lelystad (NL); Rijksuniversiteit te Groningen, 9712 CP Groningen (NL)
(72) Inventor: Kruijer, Wiebe, 3831CE Leusden (NL)
(74) Representative: Prins, Adrianus Willem

(57) **Abstract**

The invention relates to the field of embryology, embryogenesis, molecular genetics, (veterinary) medicine and zoo-technical sciences, and to the generation of stem cell-like cells. The invention provides a method for obtaining a stem cell-like cell from a sample taken from a multicellular organism, preferably an organism with some measure of differentiated tissue, thus preferably being beyond the morula stage, comprising culturing cells from said sample and allowing for transcription, translation or expression by at least one of said cells of a gene or gene product that in general is differentially expressed at the various different phases of embryonic development of the organism as described above.

## Description

The invention relates to the field of embryology, embryogenesis, molecular genetics, (veterinary) medicine and zoo-technical sciences, and to the generation of stem cell-like cells.

A particular problem in embryology is the understanding of appearance, during development, of complexity of form and function where previously no such complexity existed. Historically two contrasting points of view have been held on this problem. One of these, the so-called theory of *epigenesis,* considered that during development there is actually the creation of new structures; whereas the other, the theory of *preformation,* maintained that a pre-existing diversity is already present in the fertilized egg (or in the sperm) and that future development consists merely in the unfolding and rendering visible of this innate diversity. The embryological investigation of the past hundred-and-fifty years have demonstrated most conclusively that the actual processes of development are of an epigenetic nature but the doctrine of preformation has been reintroduced, in a much modified form, in the explanation of the facts established by modern genetics.

Classically, the fundamental morphogenetic mechanisms during embryonic development are described under the headings of *growth, differentiation* and *metabolism.* Growth is increase in spatial dimensions and in weight; it may be *multiplicative* (increase in number of nuclei and (or) cells), *auxetic* or *intussusceptive* (increase in the size of cells) or *accretionary* (increase in the amount of non-living structural matter). Differentiation is seen as an increase in *complexity* and *organization.* This increase may be in the number of variety of cells and may not at first be apparent ("invisible" differentiation, e.g. determination of fates, segregation of potencies, but, when apparent ("visible" or "manifest" differentiation), constitutes *histogenesis,* the formation of differentiated or somatic tissue. Metabolism includes the chemical changes in the developing organism.

In the normal development of an embryo these fundamental ontogenetic processes are all closely interlinked, constituting an integrated system. They fit in with each other in such a way that the final product comes into being by means of a precise co-operation of reactions and events.

From a descriptive point of view the principal stages in embryological development leading to the formation of differentiated tissue within the developing organism can be described in stages. A first phase comprises maturation. The process associated with maturation of the female and male germ cells (gametes) including the reduction (meiotic) division. The mature gametes (ova in the female, spermatozoa in the male) are highly specialized cells which when fully differentiated do not usually live long unless they take part in fertilization.

Fertilization is the fusion of a female and a male gamete which results in the formation of the zygote or fertilized ovum. The zygote, although resulting from the fusion of two highly specialized cells, is typically regarded as being the most unspecialized (undifferentiated) of all metazoan cells, being the totipotent, pluripotent or stem cell from which a new individual and differentiated somatic (adult) tissue can develop. The process of differentiation, comprises forward differentiation from this toti- or pluripotent cell to specialized cells that have much more restricted potency but also transdifferentiation is observed where cells with distinct characteristics develop into cells with other distinct characteristics.

After fertilization the zygote soon undergoes repeated subdivision or cleavage by mitosis so that a number of cells, *blastomeres,* each much smaller than the ovum itself, is produced. After a certain number of cell divisions (generally at the 8-16 cell stage), the developing organism is called a morula. Typically, the individual cells of a morula, or at least the greater part of it, are still pluripotent, separation of cells from the morula stage can lead to the production of several new (cloned) individuals, stemming from one zygote, and at the morula stage the organism is in general seen as mainly comprising undifferentiated tissue. However, at some time at the end of cleavage the blastomeres are eventually grouped to form a hollow sphere of cells, the blastula or, in mammals, the blastocyst. In this blastula stage, the organism is in general seen as comprising for the first time some measure of differentiated tissue, whereby totipotent or pluripotent cells can mostly be found in the inner cell mass (ICM) of the blastula. Most of these cells also differentiate further, some of these become the so-called adult stem cells, but most differentiate into the specialized cell type to which they are being destined. A subgoup of stem cells, also called primordial germ cells, are kept in stock for the formation of gametes for a future generation. Experiments on, and intravitam staining of the blastulae of lower vertebrates, especially Amphibia, have made it possible to delimit the future fated of all regions of the blastula and thus to ascertain their potency, i.e., what localized areas become in normal development. The different areas of the blastula can thus be referred to as presumptive organ regions, e.g. one region is presumptive notochord, another presumptive neural plate, etc. It is the blastocyst, which in mammals proceeds to implantation in the uterus.

The blastula stage is succeeded by the gastrula which results from changes in position and displacements (*morphogenetic movements)* of the various presumptive regions of the blastula. In normal development the process of formation of the gastrula, or *gastrulation,* results in certain surface regions of the blastula becoming invaginated within the blastular cavity to form the endoderm, notochord and mesoderm, the tissue undergoes visible differentiation. The region through which invagination occurs is called the *blastopore.* The cells which remain on the blastular surface constitute the *ectoderm,* from which the *epidermis* and the *neural plate* are derived, and by their expansion and multiplication they gradually replace the areas of presumptive endoderm, notochord and mesoderm as the latter are invaginated. Gastrulation results in the establishment of the three primary germ layers, *endoderm, mesoderm* and *ectoderm,* again comprising, albeit somewhat mere differentiated, stem cells, and brings the presumptive organs of the embryo into the positions in which they will undergo their subsequent development. In reptiles, birds and mammals this gastrulation period is represented by the embryonic disc and primitive streak stages.

The gastrula stage is followed by the neurula stage in which the neural plate and the axial embryonic structures are elaborated. In Amphibia this is known as the neurula. This stage corresponds roughly to the somite stages in human development. At the end of the neurula, or somite stage of development the general pattern of the embryo is well established and later embryos are said to be in the so-called functional period of development.

The earlier embryonic stages, which have been described above, result in the appearance of the general embryonic pattern before the onset of specific function in the primordia of the different organs and tissues which are differentiated in these stages. Functions in the general sense are carried out at all times as all the cells are undergoing metabolic changes and must "work to live". But with the onset of specific functions such as beating of the heart, contraction of muscles, secretion by glands, etc., the embryo enters on what may be called the functional period of development. Different organs, of course, commence to function at different times and no sharp distinction can be made between *pre-functional* and *functional* stages; growth and differentiation proceed in both. Nevertheless it is useful to consider the process of earlier stages as blocking out the main embryonic organ systems which will subsequently be elaborated under the influence of the specific functions which they perform. The functional influence does not, by any means, replace the genetically determined general pattern of development, but, in the development of many organs and tissues (e.g., the heart and blood vessels and the skeletal system), the effect of the function of an organ on its development is considerable. The functional stage of development results in the different organs and tissues coming into physiological relationship with each other and, therefore, in a degree of integration of total function which cannot exist in earlier stages.

The integration is facilitated, and indeed rendered possible, by the differentiation of the vascular and nervous systems and the onset of function in the endocrine glands. When the developing organism has entered such a functionally differentiated stage in its development, most if not all requirements underlying further developments in the following foetal, post-natal or adult stages of the individual have been met.

In short, all multicellular organisms are in general formed from a single totipotent stem cell, the embryonic stem (ES) cell. ES cells are clonal cells, for example derivable from the inner cell mass of an developing blastula and are capable of adopting all the cell fates in a developing embryo. They form the pluripotent cells of the inner cell mass of mammalian pre-implantation embryos. These cells can in general be isolated and maintained *in-vitro* as toti- or pluripotent cells or stem cell-like cells, and now can even give rise to new (cloned) individuals (see also http://www.nuffieldfoundation.org/bioethics).

For example, genetically modified mouse stem cells have been cultured together with feeder cells (such as mouse ES cells or embroid bodies) to induce differentiation. However, to isolate or study the fate of stem cells, also under different inductive environments a cumbersome selection procedure is required. Such a selection method generally involves modification of stem cells by genetic means. Stem cells for example need essentially be modified to express a resistance marker (for example a neomycin resistance) gene in order to eventually eliminate feeder cells and select specifically for stem cells. For this technology to have application in medicine or even for zoo-technical applications, the use of a selection system based on the detection of a resistance marker is not desirable.

ES cells in general have an indefinite life span and can proliferate extensively when propagated under appropriate culture conditions involving the use of embryo-derived feeder cells or media containing lymphocyte inhibitory factor (LIF) (e.g in the case of mouse ES cells). When for example injected into mouse blastocysts, *in-vitro* propagated totipotent or pluripotent embryonic stem cells can contribute to all tissues of the recipient embryo as well as to the germ line and be a source of new (cloned) individuals. In addition, these pluripotent embryonic stem cells can be induced to differentiate *in-vitro* yielding differentiated derivatives representative of all three germ layers including neuronal, myocardial and endothelial cells.

Functionally differentiated organisms with differentiated tissues such as mesodermal, ectodermal, endodermal, or even adult or somatic tissues may still contain a variety of cells that have normal functions in tissue such as the continuous generation of new cells, also in response to injury and aging (i.e cell renewal). Such "differentiated or adult stem cells" have for example been found in bone marrow, bone marrow stroma, muscle and brain. Adult somatic stem cells have similar, albeit some restricted capacity of self-renewal and may give rise to daughter cells with the same potential as well as daughter cells with a more restricted differentiation capacity. The differentiation potential of stem cells in differentiated tissues is in general thought to be limited to cell lineages present in the organ from which they were derived, excluding of course the potential of those primordial germ cells that are required for gamete formation.

The invention provides a method for obtaining a stem cell-like cell from a sample taken from a multicellular organism, preferably an organism with some measure of differentiated tissue, thus preferably being beyond the morula stage, comprising culturing cells from said sample and allowing for transcription, translation or expression by at least one of said cells of a gene or gene product that in general is differentially expressed at the various different phases of embryonic development of the organism as described above. Hereby the invention provides for a dedifferentiation and/or selection of at least one or some cells from said sample for stem cell-like cell characteristics based on for example the transcription (and possible further translation) of distinct gene products or the presence of distinct transcription factors (detectable by for example detecting relevant promotor activity or detecting other relevant gene products such as mRNA or (poly)peptides derived from a gene that is for example differentially expressed at the morula stage versus the neural stage, or the blastula stage versus the functional stage, or the blastula stage versus the adult stage. By allowing the cells from an already forward differentiated or specialised tissue to again or preferentially transcribe early phase genes that are no longer transcribed, or alternatively, to suppress or down-regulate transcription of later phase genes, said dedifferentiation or selection of a stem cell-like cell is provided, even from an already functionally differentiated organism as described above. The invention thus provides a dedifferentiated stem cell or stem cell-like cell, and cell cultures or cell lineages derived thereof. The pluripotency of stem cell-like hES-eq cells has been established by production of teratomas following transplantation in immunodeficient (SCID) mice, and by injection of hES-eq cells in mouse blastocyst and assessment of the level of chimerism based on (i) detection of human or isogenic cell surface marker; (ii) the expression of human genes in developing tissues using RT-PCR and human gene-specific primers and (iii) expression of a hES-eq expressed reporter (GFP, LUC, LacZ) following stable or transient expression of this gene in hES-eq cells -injection of hES-eq cells into the amniotic cavity of chick stage 4 embryo's and detection of chimerism in the developing tissues by analysis of cell surface marker expression and RT-PCR using human gene-specific primers.

Totipotent dedifferentiated stem cells as provided herein are obtained from for example human tissue. These hES-eq(uivalent) cells are characterised by expression of the stem cell-specific transcription factors Oct4, Sox2 and UTF1, specific pattern of expression of the cell surface markers stage-specific embryonic antigens SSEA-1 and SSEA-3, TRA-1-60 and TRA-1-81 and alkaline phosphatase, specific gene expression profiles as determined by DNA gene expression micro-arrays, and the capacity to form differentiated derivatives from embroid bodies following aggregation in the presence of retinoic acid or DMSO and expression of cell lineage markers (depending of the treatment) Troma-1 (endoderm derivatives), Neurofilament type-1 (neuronal derivatives), Cardiac Myosin Heavy chain (cardiac muscle), expression of telomerase activity and normal karyotype corresponding with the sex of the donor. From other mammals, similar ES-cells were obtained demonstrating similar (species-specific) characteristics.

Other procedures provided herein and allowing facilitating the recovery of dedifferentiated stem cells or stem cell-like cells involve manipulation of gene expression affecting cell cycle progression in the G0-G1 phase of the cycle including pre-treatment with transforming growth factors for transient induction of cell cycle arrest, or timed addition of extracellular factors that block differentiation at early stages of development i.e. nodal to antagonize BMP effects and removal of differentiating agents from Fetal Calf Serum (FCS) containing media, or timed addition of extracellular factors that sustain the proliferation of ES-like cells i.e. LIF, growth factors including FGF's, PDGF's and interleukins, or co-culture of selectable adult stem cells as indicated above with pluripotent ES cells (mammalian, human, primate) classified as such; human embryonal carcinoma cells classified as such i.e. N-tera-2; yolk-sac tumor cell lines classified as such, or culture of somatic stem cells with conditioned medium derived from the above cells lines, or isolated factor(s) present in the conditioned medium of the cell lines indicated above. In the case of the production of dedifferentiated stem cells from humans, tissue samples were obtained under condition of (patient) informed consent and experiments involved controlled laboratory processes. The isolation of human ES equivalent (hES-eq) cells from pre- and post-natal and adult human tissue may further involve the following isolation and selection of hES-eq cells on the basis of expression of Oct4 promoter driven cell surface marker(s) (Schoorlemmer et al., Mol. Cell. Biol. 14:1122-1136, 1994) allowing specific recognition of the cell surface expressed molecules by specific antibodies. Isolation procedures may involve separation of hES-eq cells with magnetic bead or fluorescence activated cell sorting (FACS). Oct4-promoter driven expression of Green Fluorescent Protein (GFP) and isolation of GFP-expressing cell by FACS is provided as well Oct4-promoter driven expression of the neomycin resistance gene and selection of G418 resistant cells is optional.

Such a dedifferentiated stem cell, or stem cell-like cell, as provided herein can be used for all purposes that seem fit as use for stem cells in general, but offer also distinct advantages beyond current available stem cells. In one specific embodiment, it is now provided to take a sample from an individual suffering from a disease (in particular helpful, when the organism is human, in human medicine for for example bone-marrow deficient patients, patients suffering from Parkinson disease or Alszheimers disease or diabetes or other disease where suppletion or transplantation with specialised cells is contemplated) or otherwise in need of transplant treatment, treat the sample as described herein, obtain a dedifferentiated stem cell from said individual, grow it into a culture of stem cell-like cells, provide, if required, for forward differentiation of said culture towards a more differentiated or required specialised cell type, and use such a culture or parts thereof as graft for treatment of the exact same individual. No major adverse immune response are to be expected when the graft is, so to speak, put back into the individual, however now provided with desired functionality's deemed necessary for treatment. In particular, said cells need no recombinant engineering to provide an immunological match with the recipient, the recipient being also the donor of the cells to begin with. In other words, the recipient can be his or her own donor.

A cell as provided herein can also be used to grow distinct tissue types, such as (heart) muscle cells, blood cells, blood vessel cells, cartilage or bone tissue, neural cells, skeletal tissue, and so on, for which, again no immunological match is required when placed back into the donor/provider of the source of the graft. Of course, these cells lend themselves also to the provision of said immunological matches in case other recipients are contemplated, using methods known in the art and classical employed with the embryonic stem cells that give rise to specialised tissue or cells.

Of course, a cell as provided herein finds its use also in providing new (cloned) individuals, which is in particular advantageous, when the organism is a vertebrate such as a fish (salmon, trout, eel), poultry (chicken) or mammalian (mice, rats, guinea pigs, or other small laboratory animals, or farm animals like ruminants or pigs) in the field of the creation of (near identical or cloned) experimental animals or farm-animals or animals for the production of xenotransplant-tissue (often pigs are used) or other desired (recombinant) products. Again, a major advantage of the method as provided herein lays in the fact that such cells can now be obtained from functionally differentiated or even specialised adult somatic tissue, such as muscle, brain, blood, none marrow, liver, mammary gland, and so on, allowing to first select the desired animal from amongst other related but less desirable animals (e.g. on production characteristics), and than cloning it, using for example an easily obtainable tissue biopsy as sample for the provision of the desired stem cell-like cell or cells from which cloning can commence. Such dedifferentiated cells as provided herein can, if required in an intermediate step, be injected into a (if desirable an unrelated) developing embryo (preferably blastula stage) and develop into a chimeric organism from which primordial germ cells or gametes with the desired specificity can be harvested, but can also be used for direct embryonic development.

In a preferred embodiment, a method according to the invention is provided wherein said cells from said sample are cultured in the relative absence of a differentiation factor such as different members of the steroid-hormone receptor superfamily
[(nuclear receptors): ARP-1, RAR (retinoic acid receptor)] but preferably in the relative absence of retinoid or retinoic acid or analogue thereof. This allows for the differential expression of differentiation factor or retinoic acid induced or suppressed genes or fragments thereof. Culture medium can be deprived of retinoids or retinoid activity by for example charcoal filtration of the medium itself or its constituting components such as the serum (preferably bovine calf serum, preferably free of specified pathogens is used), measuring resulting activity and using the medium sufficiently deprived of said activity. Growth media are otherwise produced and used as known in the art of cell culture, in particular of stem cell culture.

The invention also provides further comprising a selection method preferably not based on the genetic modification of somatic cells for the identification of embryonic stem cell like cells from amongst a population of cells in adult somatic tissue. Dedifferentiated adult somatic cells which have this broad differentiation repertoire are herein also referred to as a somatic stem cell embryonic stem cell equivalent (SSCES-eq) or stem cell-like cell. SSCES-eq have characteristics that are very similar or even indistinguishable from embryo-derived cells (ES) and have a developmental repertoire that is close or even identical to that of ES cells. This system is based on the observation that specific marker genes such as the well known transcription factor Oct 4 are differentially expressed during the developmental processes observed in the growing embryo.

These de-differentiated SSCES-eq can be multiplied *in vitro* and can under the right circumstances give rise to an almost unlimited source of stem cells to be used in a variety of ways. Dedifferentiated somatic stem cells from a single donor can be made recipient-independent and broad range applicable by genetic inactivation *in vitro* of the MHC locus. Therefore this invention provides the means to treat more easily individual patients, in a strict donor-recipient relationship, with SSCES-eq cells derived from their own tissues with properties equivalent to ES cells specialised for a given task. Furthermore it provides the means to treat various diseases in different affected individuals with general source of de-differentiated SSCES-eq cells. The invention shows that adult somatic stem cells, although more specialised than pluripotent ES-cells can be used as alternative source for embryo-derived ES-cells, for the purpose of repairing or replacing body tissues (for example blood, nerve and myocardial tissues), with the main advantage that immunological matching is nor required.

The present invention provides a method for *in-vitro* selecting a somatic stem cell like cell from differentiated tissue material or samples comprising culturing cells from said material under conditions allowing for induction of expression of essential pre-implantation (early blastocyte stage in mammals) gene products and/or suppression of expression of non-essential pre-implantation gene products. A somatic stem cell or tissue herein refers to any differentiated 'body' cell or tissue be it of mesodermal, endodermal or ectodermal descent ( for example blood, immune system, nerve, myocardial, muscle, intestinal tissue). Further the invention provides a method for *in-vitro* selecting a somatic stem cell like cell from post-implantation material comprising culturing cells from said material under conditions allowing for induction of expression of essential post-implantation gene products and/or suppression of expression of non-essential pre-implantation gene products.
The invention provides a method of selection of a stem cell like cell (SSCES-eq) based on detecting differences in gene expression patterns between genes differentially expressed at different stages of embronic development, in mammals for example identifiable as pre- and post-implantation stages. Methods to detect differential gene expression patterns are known in the art and comprises methods aimed at detecting 'nucleic acid' and/or 'amino acid'. 'Nucleic acid' herein refers to an oligonucleotide, nucleotide or polynucleotide, and fragments or portions thereof, and to DNA or RNA of genomic or synthetic origin which may be single- or double-stranded, and represents the sense or antisense strand. 'Amino acid' herein refers also to peptide or protein sequence. Included in the scope of the invention is detection of different alleles of the polypeptide encoded by nucleic acid sequences or gene of interest. As used herein, an 'allele' or 'allelic sequence' is an alternative form of a polypeptide. Alleles result from a mutation [eg. a change in the nucleic acid sequence, and generally produce altered mRNA or polypeptide whose structure or function may or may not be altered]. Any given polypeptide may have none, or more allelic forms. Common allelic changes that give rise to alleles are generally ascribed to natural deletions, additions or substitutions of amino acids. Each of these types of changes may occur alone, or in combination with the others, one or more times in a given sequence. Deliberate amino acid substitution may be made on the basis of similarity in polarity, charge, solubility, hydrophobicity, and/or the amphipathetic nature of the residues as long as the biological activity of the polypeptide is retained. A 'deletion' is defined as a change in either nucleotide or amino acid sequence in which one or more nucleotides or amino acid residues, respectively, are absent. An 'insertion' or 'addition' is that change in nucleotide or amino acid sequence which has resulted in the addition of one or more nucleotides or amino acid residues, respectively, as compared to the naturally occurring polypeptide(s). A 'substitution' results from the replacement of one or more nucleotides or amino acids by different nucleotides or amino acids, respectively. Included is a polypetide variant. A 'variant' of a polypeptide is defined as an amino acid sequence that is different by one or more amino acid 'substitutions'. A variant may have 'conservative' changes, wherein a substituted amino acid has similar structural or chemical properties eg replacement of leucine with isoleucine. More rarely a variant may have 'non-conservative' changes (eg replacement of a glycine with a tryptophan). Similar minor variations may also include amino acid deletions or insertions, or both.

Methods to detect differential gene expression patterns are known to those skilled in the art. These procedures include, but are not limited to DNA-DNA or DNA-RNA hybridisation. The form of such quantitative methods may include, Southern or Northern analysis, dot/slot blot or other membrane based technologies; PCR technologies such as DNA Chip, Taqman®, NASBA, SDA, TMA, *in-situ*-hybridisation, protein bioassay or immunoassay techniques ELISA, IFA and proteomic technologies. Other evolving technologies such as 'metabolomics' can be employed to look at changes in metabolic profiles between tissues and/or cell types.

The invention also provides a method of selection whereby said material is derived from vertebrate tissue and/or cells. The term vertebrate refers to a higher life form having a spinal column. One preferred embodiment is that the somatic stem cell like cell (SSCES-eq) selected is from adult somatic tissue. Included in the scope of the invention is somatic stem cells obtainable from primate somatic tissues. 'Tissue' herein refers to a collection or aggregate of individual cell types. The invention further provides a method of selection whereby said tissue comprises muscle tissue and/or bone marrow tissue and/or bone marrow stroma and/or nerve tissue and/or brain tissue and/or blood. Unlike ES cells the dedifferentiated stem cells as provided herein can be derived from a multitude of tissue types. These cells are also derivable from bone marrow, bone marrow stroma, muscle and brain tissue. The invention further provides a method of selection whereby said tissue is adult tissue. One preferred embodiment is that the somatic stem cell like cell is obtainable from nerve tissue and/or bone marrow tissue and/or bone marrow stroma and/or muscle tissue and/or brain tissue and/or blood, more preferably human. The invention further provides a method of selection comprising selecting said cell from the ectoderm and/or mesoderm and/or endoderm layer of said tissue. The invention further provides a method of selection comprising selecting said cell by detection of expression of cell-specific transcription factors Oct4 and/or Sox2 and/or UTF1 or homologues or orthologues thereof. The tissue-specific transcription factors Oct4 and/or Sox2 and/or UTF1 are required for the development of a precise somatic stem cell lineage. Hence the dedifferentiated stem cell provided herein can be identified on the basis of expression of these stem cell-specific transcription factors. The definition 'homologue' is a term for a functional equivalent. It means that a particular subject sequence varies from the reference sequence by one or more substitutions, deletions, or additions resulting in 'amino acid' that encode the same or are functionally equivalent, the net effect of which does not result in an adverse functional dissimilarity between the reference and the subject sequence. Orthologues are similar genes found with other species.

The invention further provides a method further comprising selecting said cell by detection of expression of cell surface markers stage specific embryonic antigens SSEA-1 and/or SSEA-3 and/or TRA-1-60 and/or TRA-1-81 and/or alkaline phosphatase or analogs thereof. The invention further provides a method of selection whereby said somatic stem cell like cell has telomerase activity. The invention further provides an isolated somatic stem cell obtainable from adult somatic tissue having telomerase activity. 'Telomerase' activity herein refers to the activity of a specific enzyme termed telomere terminal transferase which is involved in the formation of telomere DNA. Telomers are required for replication and stable inheritance of chromosomes. The invention further provides a method of selection whereby said somatic stem cell like cell has trans-differentiation capacity. Adult somatic stem cells have a high trans-differentiation capacity. By trans-differentiation capacity it is meant that somatic stem cells have the capacity of indefinite self-renewal by producing a multitude of daughter cells through repeated divisions. They give rise daughter cells with the same potential, as well as daughter cells with a more restricted differentiation capacity. Neuronal stem cells can give rise to blood cells after transplantation into the blood of irradiated stains of mice. Also muscle progenitor cells have been shown to be capable of trans differentiation into blood. Furthermore, bone marrow stroma cells transplanted to the brain can generate astrocytes and hematopoietic stem cells can give rise to myocytes. The invention further provides a somatic stem cell like cell comprising recombinant nucleic acid. Classically, ES cells are seen as extremely useful for creating transgenic animals, the the dedifferentiated stem cell as provided herein is equally suitable. Methods to transduce stem cells are known in the art for example by using a gene delivery vehicle. 'A gene delivery vehicle' herein is used as a term for a recombinant virus particle or the nucleic acid within such a particle, or the vector itself, wherein the vector comprises the nucleic acid to be delivered to the target cell and is further provided with a means to enter said cell.

The invention provides an isolated stem cell like cell obtainable through selection capable of clinical use. A dedifferentiated stem cell or cells from a single donor can be made recipient-independent and broad range applicable by genetic inactivation *in vitro* of the MHC locus. Included in the scope of the invention is a pharmaceutical composition comprising a somatic stem cell like cell or culture transduced with a gene delivery vehicle, to generate different tissue types for application in gene therapy. One usage is the generation of different types of tissue and/or tissue renewal. Another is the repair and/or replacement of different types of tissue. These tissues derived from somatic stem cell like cells could be administered to a patient by transplantation into host tissue or by grafting for use in the treatment of by way of example Parkinson disease and/or cardiovascular disease and/or liver disease. For example adult somatic stem cells or the dedifferentiated pluripotent ES-like cells can be obtained for muscle tissue or cordial blood of a given patient donor, dedifferentiated (multiplied) *in-vitro*, and can then be applied for brain tissue repair as donor recipient. This source of stem cells can be used in any kind of tissue renewal and/or repair and/or replacement in cases such as, but not limited to Parkinson disease, cardiovascular diseases, liver disease. Another preferred embodiment of the invention relates to the production of non donor-specific pluripotent ES-like cells for use in non-donor recipient tissue repair and/or renewal and/or replacement treatments. Dedifferentiated somatic stem cells from a single donor can be made recipient-independent and broad range applicable by genetic inactivation in *vitro* of the MHC locus. This has an advantage in that a general source of human stem cells can be applied for tissue repair and/or renewal and/or replacement treatments without tissue rejection problems arising. The invention further provides use of a cell or culture or graft or animal as provided herein in studying the biology of vertebrate development, in transplantation, in drug screening and drug discovery and in cosmetic surgery, whereby again immunological mismatches can be avoided. Included in the scope of the invention are cross species recipients.

Oct 4 (Genbank accesion S68053) is one of the mammalian POU transcription factors expressed by early embryo cells and germ cells. It is a marker for PGCs and pluripotent stem cells in mice. The activity of OCT4 is essential for the identity of the totipotent founder cell population in the mammalian embryo. Oct 4 determines paracrine growth factor signaling from stem cells to the trophectoderm. Oct-4 is a transcription factor whose expression is associated with an undifferentiated cell phenotype in an early mouse embryo and is down-regulated when such cells differentiate. Expression of oct-4 in embryonic stem cells is controlled by a distal upstream stem cell specific enhancer that is deactivated during retinoid or retinoic acid (RA) induced differentiation by an indirect mechanism in general not involving binding of RA receptors. Said enhancer is thought to contain no retinoic acid receptor (RA) binding sites. Oct 4 is subject to negative regulation by other differentiation factors such as different members of the steroid-hormone receptor superfamily (nuclear receptors). ARP-1, RAR (retinoic acid receptor). It has been shown that negative regulation of OCT4 expression during RA induced differentiation of embryonic stem cells is controlled by two different mechanisms, including deactivation of the stem cell specific enhancer and by promoter silencing by orphan hormone receptors.

### Protocol for charcoal stripped serum to remove retinoids

Day 1: Prepare dextran-coated charcoal suspension
   Add 450 ml of tissue quality H2O to 50 ml of TRIS/HCL 0.1M, pH 8.0 Dissolve in this buffer 0.25 gram of Dextran T500 Pharmacia, nr. 17.0320.01 Add 2.5 grams of activated charcoal Fluka cat.nr. 05120 or Sigma C-5260 Stirr overnight at 4°C in thightly locked vessel
Day 2
   Heat 200 ml of Fetal Calf Serum (FCS) for 30 minutes at 56°C in a water bath.
   In the meantime fill 12 x 50 ml (plastic) centrifuge tubes with the charcoal suspension.
   Spin in swing-out rotor, 20 min, 1000 x g.
   Discard the supernatant. Remove as much fluid as possible without touching the pellets.

Add the serum to 6 charcoal pellets and resuspend the pellets in the serum.
Incubate in a water bath at 45° for 45 min, while shaking.
Spin in swing-out rotor, 20 min, 1000 x g.

Add the serum supernatant to the 6 remaining charcoal pellets and repeat the whole procedure.

Add the serum to 6 clean centrifuge tubes and spin again.
Pool the serum in a clean bottle and filter/sterilize before freezing.
Retinoids or retinol derivatives are for example all-trans-*retinyl* esters, all-trans-retinol, 3,4-didehydro-retinol, 4-oxo-retinol, all-trans-retinal, 4-oxo-retinal, beta-carotene, all-trans-retinoic acid, 18-hydroxy-retinoic acid, 4-hydroxy-retinoic acid, 4-oxo-retinoic acid, 9-cis-retinoic acid, or 9-cis-4-oxo-retinoic acid.

### Dedifferentiation experiments

### mouse

Hematopoietic stem cells isolated from embryonic liver of B6.SJL-Ly5.1 and eGFP transgenic mice are purified by Fluorescent Activated Cell Sorting (FACS). Hematopoietic stem cells are collected on the basis of Ly5.1 expression. Pools of approximately 100 cells are injected in blastocysts of congenic C57BL/6 strain of mice expressing the Ly5.2 allele. Injected blastocysts are disaggregated and Ly5.1 expressing cells are isolated by FACS and collected as single cells. Sorted cells are collected and maintained in media containing LIF and/or the MEK inhibitor. Oct4 expression is determined by single cell RT-PCR using murine Oct-4-specific oligonucleotide primers.

Alternatively, mouse liver hematopoietic stem cells are mixed with microsurgically dissected inner cell mass cells of blastocysts followed by the procedures described above.

### human

Hematopoietic stem cells (CD34 positive) are injected into C57BL/6 or immunodeficient NOD-SCID or Rag-/- mouse blastocysts and expression of human Oct4 is determined by RT-PCR.
Blastocysts injected with CD34-positive human hematopoietic cells transduced with Oct-4 promoter-eGFP fusion genes are assayed for eGFP and isolated by FACS.

FACS sorted cells are maintained in LIF and MEK inhibitor containing media.

## Claims

1. A method for obtaining a stem cell-like cell from a sample taken from a multicellular organism comprising culturing cells from said sample and allowing for transcription, translation or expression by at least one of said cells of a gene or fragment thereof that in general is differentially expressed at different phases of embryonic development.

2. A method according to claim 1 wherein said organism is functionally differentiated .

3. A method according to claim 1 or 2 wherein said organism comprises a vertebrate.

4. A method according to anyone of claims 1 to 3 comprising culturing cells from said sample in the relative absence of a differentiation factor

5. A method according to claim 4 wherein said factor comprises retinoid activity.

6. A method according to anyone of claims 1 to 5 wherein said gene is overexpressed in an early phase of embryonic development.

7. A method according to claim 6 wherein said early phase comprises the blastula stage.

8. A method according to claim 7 wherein, in mammals, said blastula stage comprises a pre-implantation stage.

9. A method according to anyone of claims 1 to 8 wherein said gene comprises Oct 4 or orthologue thereof.

10. A method according to anyone of claims 1-9 further comprising selecting said stem cell-like cell by detection of expression of cell surface markers stage specific embryonic antigen.

11. A method according to claim 10 wherein said antigen comprises SSEA-1, SSEA-3, TRA-1-60 , TRA-1-81 and/or alkaline phosphatase or analogue thereof.

12. A dedifferentiated stem cell.

13. A stem cell like cell obtainable by a method according to anyone of claims 1-11.

14. A cell according to claim 12 or 13 comprising a recombinant nucleic acid.

15. A culture comprising a cell according to claim 12 to 14.

16. A graft or transplantation material comprising a cell according to anyone of claims 12 to 14 or a culture according to claim 15.

17. An animal comprising a cell according to anyone of claims 12 to 14 or a culture according to claim 15.

18. Use of a cell according to anyone of claims 12 to 14 or a culture according to claim 15.

19. Use according to claim 18 for the preparation of a pharmaceutical composition.

20. Use according to claim 19 for the preparation of a pharmaceutical composition for the treatment of an individual with a graft.

21. Use according to claim 20 wherein said individual or a sample thereof comprises a source of said graft.

22. Use according to claim 18 for cloning of an animal.

23. Use according to claim 22 wherein said animal comprises an experimental animal, or a farm animal or an animal for xenotransplant production.
